# EUROPEAN PATENT APPLICATION

(11) **EP 2 837 612 A1**
(43) Date of publication of application: **18.02.2015**
(21) Application number: 13180172.2
(22) Date of filing: 13.08.2013
(51) Int. Cl.: C07B 43/04, C07C 209/36

(54) **Nitrogen-doped carbon nanotubes as high selective noble metal-free catalysts for hydrogenation of functionalized nitroaromatics**

(71) Applicant: Bayer Technology Services GmbH, 51368 Leverkusen (DE)
(72) Inventor: Dr. Lu, Huachang, 50858 Köln (DE); Dr. Schlüter, Oliver Felix-Karl, 51381 Leverkusen (DE); Prof. Dr. Mleczko, Leslaw, 41542 Dormagen (DE); Buchholz, Sigurd, 50762 Köln (DE)
(74) Representative: BIP Patents

(57) **Abstract**

The invention relates to a process for hydrogenating halogen-substituted nitro-aromatic compounds or functional nitro-aromatic compounds using nitrogendoped carbon nanotubes (NCNTs).

## Description

Functionalized aniline derivatives and halogen-substituted anilines are important industrial intermediates in the synthesis of many agrochemicals, organic dyes, pigments, herbicides, pesticides and pharmaceuticals, etc. The hydrogenation of functional group-substituted nitro aromatic molecules and halogen-substituted nitro aromatic molecules, such as halogen, carbon-carbon double bond, nitrile functionalizes nitro aromatics, is a common strategy for the production of the corresponding functionalized (halo) aniline derivatives. It remains a challenging task for both academic research and industrial application to chemoselectively hydrogenate nitro groups in the presence of halogen substituents and other functional substituents (H.-U. Blaser, H. Steiner and M. Studer, ChemCatChem 2009, 1, 210-221*;* A. Corma, P. Serna, et. al., J. Am. Chem. Soc. 2008, 130, 8748-8753*;* G. Wu, M. Huang, et. al., Synthesis 2003, 11, 1657-1660*).* The traditional and most often used catalyst systems for such selective hydrogenation of nitro groups are the noble metals Pt and Pd, mostly supported on alumina, titania, MgO, iron oxides or active carbon, as well as the classical Raney nickel (RaNi) or supported Ni (C. Xiao, X. Wang, et. al., Current Organic Chemistry 2012, 16, 280-296). Low selectivity due to the loss or reduction of functional groups in the process of hydrogenation over these mentioned platinum or nickel catalysts is frequently observed. In addition, dehalogenation of the haloaromatics in the process of hydrogenation over these mentioned platinum or nickel catalysts is frequently observed. On the other hand, the high cost of noble metal due to the scarcity limits of the large scale industrial application. Furthermore, the metal leaching issue, which leaves metal impurities in the final halo aniline products and brings extra post-synthesis purification costs as well as negative environmental impact. Therefore it is important to develop an economically feasible catalyst with high selectivity, and hence low dehalogenation rate in the case of halogen-substituted anilines, for such hydrogenation reaction.

The incorporation of heteroatoms like nitrogen atoms within the carbon nanotubes (CNT) network will generate some structural defects in the nanotubes due to the different atom size. On the other hand, the introduction of foreign atoms will also dramatically influence the physical, chemical and electronic properties of these nanotubes. In particular, the substitutional doping of nitrogen has brought significant changes in the mechanical hardness, electrical conductivity, and chemical reactivity of CNTs (P. Ayala, R. Arenal, et. al., Carbon 2010, 48, 575-586*;* L. F. Mabena, S. S. Ray, et. al., Appl Nanosci 2011, 1, 67-77*;* Z. Chen, D. Higgins, et. al., Carbon 2010, 48, 3057-3065; K. Gong, L. Dai, et. al., Science 2009, 323, 760-764*.* All these unique changes make NCNT an excellent candidate for the application fields as electronic devices, and catalysts for various reactions. It was reported that NCNTs have been used as catalyst support (L. F. Mabena, S. S. Ray, et. al., Appl Nanosci 2011, 1, 67-77*),* as well as catalyst for oxygen reduction reaction (Z. Chen, D. Higgins, et. al., Carbon 2010, 48, 3057-3065*;* K. Gong, L. Dai, et. al., Science 2009, 323, 760-764), liquid-phase C=C bond hydrogenation (J. Amadou, K. Chizari, et. al., Catalysis Today 2008, 138, 62-68*)* and nitro group hydrogenation (WO2009149849 A1).

WO 2009080204 A1 and WO 2009149849 A1 disclose the incorporation of nitrogen atoms during synthesis of the carbon nanotube by introducing some nitrogen precursors. The NCNTs synthesized in this way have a nitrogen content varying from 0.1 to 18 wt % and there are four main types of bonding configurations for nitrogen in graphitic networks. These four most common nitrogen species are pyridine-like, pyrrole-like, types of quarternary and oxidised pyridinic species (WO 2009080204 A1).

Thus far, it has not yet been reported to use nitrogen-doped carbon nanotubes (NCNTs) as catalysts for the selective hydrogenation of functional nitro aromatics and halogen-substituted nitro aromatics.

It has therefore been the problem of the invention to provide a process of using nitrogen-doped carbon nanotubes (NCNTs) in chemical synthesis of substituted aromatic compounds.

The invention has solved said problem by providing a process for highly selective hydrogenation of functional or halogen-substituted nitro-aromatic compounds using nitrogen-doped carbon nanotubes (NCNT) as a noble metal free catalyst.

It has surprisingly been found that this problem can be solved in a particularly advantageous way by the process provided by the invention.

In particular, the invention provides a process for hydrogenating functional or halogen-substituted nitro-aromatic compounds by means of hydrogen, characterized in that the hydrogenation is carried out in the presence of a catalyst which comprises nitrogen-doped carbon nanotubes (NCNTs) as catalytically active component and, the proportion of nitrogen in the nitrogen-doped carbon nanotubes is in the range from 0.05 to 20% by weight in the graphitic layers and the nitrogen is present at least partly in a pyridinic arrangement.

In one embodiment of the invention, the functional or halogen-substituted nitro-aromatic compound is present in a liquid phase and the hydrogen in the present in gaseous form.

In a further embodiment of the invention, the functional or halogen-substituted nitro-aromatic compound is present in a liquid solvent.

The liquid solvent can be an organic solvent which does not comprise any hydrogenation-active functional group. The liquid solvent can also be a mixture of such organic solvents.

In a preferred embodiment of the invention, the process is carried out at temperatures in the range from 20°C to 350°C, preferably from 40°C to 110°C.

In a further embodiment of the invention, the functional or halogen-substituted nitro-aromatic compounds and the hydrogen are present in gaseous form.

In a preferred embodiment of the invention, the process is carried out in a plurality of stages, preferably in from 2 to 10, particularly preferably from 2 to 6, very particularly preferably from 2 to 5, in particular 2 or 3, reaction zones connected in series.

In a further embodiment of the invention, the catalyst used in the process contains at least 50% by weight, preferably at least 80% by weight, particularly preferably at least 95% by weight, of nitrogen-doped carbon nanotubes.

The catalyst used in the process of the invention can be present as a fixed bed. Such fixed bed can be present in the form of a structured bed in which the catalyst activity increases in the main flow direction in the reaction zone.

The functional nitro-aromatic compound that is hydrogenated with the process of the invention can be any functional nitro-aromatic compound. In a particularly preferred embodiment of the invention, said functional nitro-aromatic compound is selected from the group consisting of 4-Nitrobenzonitrile, Ethyl 4-nitrocinnmate, 4-Nitrocinnamic acid, 4-Nitrocinnamyl alcohol, 2-Chloro-4-nitrobenzonitrile, 4-Amino-3-nitrobenzonitrile, 4-Hydroxy-3-nitrobenzonitrile, 4-Bromo-3-nitrobenzonitrile, (1,4-dihydro-6-nitro)-1,4-Methanonaphthalene and 1-(2-cyclopropylethenyl)-2-nitroBenzene. This list of preferred compounds is not limiting to the type of functional nitro-aromatic compound that can be hydrogenated using the process of the invention.

The halogen-substituted nitro-aromatic compound that is hydrogenated with the process of the invention can be any halogen-substituted nitro-aromatic compound. In a particularly preferred embodiment of the invention, said halogen-substituted nitro-aromatic compound is selected from the group consisting of 4-Iodo-nitrobenzene, 4-Fluoronitrobenzene, 4-Chloronitrobenzene, 4-Bromonitrobenzene, 1-Chloro-3-nitrobenzene, 1-Bromo-4-chloro-2-nitrobenzene, 5-Chloro-2-nitroaniline, 4-Chloro-3-nitroanisole, 2-Chloro-4-nitrotoluene, 1-Bromo-4-nitrobenzene, 2-Bromo-1-tert-butyl-4-nitrobenzene, 1-Iodo-2,4-dimethyl-6-nitrobenzene, 1-Chloro-2-iodo-5-nitrobenzene and 2-Iodo-3-nitrotoluene. This list of preferred compounds is not limiting to the type of halogen-substituted nitro-aromatic compound that can be hydrogenated using the process of the invention.

The catalyst used in the process of the invention preferably comprises nitrogen-doped carbon nanotubes (NCNTs) having a nitrogen content of from 0.1% by weight to 18% by weight and particularly preferably from 0.5% by weight to 16% by weight in the graphitic layers.

The proportions of nitrogen in the nitrogen-doped carbon nanotubes are particularly advantageous because there is an optimum of the nitrogen content of the nitrogen-doped carbon nanotubes for the hydrogenation of functional or halogen-substituted nitro-aromatic compounds.

The proportion of nitrogen present in the nitrogen-doped carbon nanotubes that are used in the process of the invention can be present not only as at least a proportion of pyridinic nitrogen, but also in the form of quaternary nitrogen and/or nitro nitrogen and/or nitroso nitrogen and/or amine nitrogen.

The proportions of nitro and/or nitroso and/or amine nitrogen are of subordinate importance to the NCNTs used in the process of the invention insofar as their presence does not significantly hinder the generation of the NCNTs in advance of using them as a catalyst as long as the above-described proportions and ratios in respect of pyridinic and/or quaternary nitrogen are present.

The proportion of pyridinic nitrogen in the catalyst is preferably at least 20 mol% of the nitrogen present in the nitrogen-doped carbon nanotubes. The proportion is particularly preferably greater than 30 mol%.

Likewise, the ratio of pyridinic nitrogen to quaternary nitrogen is preferably at least 1.25 mol/mol, particularly preferably at least 1.3 mol/mol.

The proportions and ratios indicated are particularly advantageous because the pyridinic nitrogen in particular has a particularly strong influence on the catalytic activity of the catalyst for the hydrogenation of functional or halogen-substituted nitro-aromatic compounds.

The process of the invention surprisingly and advantageously allows the heterogeneously catalyzed hydrogenation of functional and halogen-substituted nitro aromatics with a high conversion, selectivity and yield and dispenses with the use of expensive noble metals or pyrophoric catalyst constituents.

The process of the invention will be illustrated below with the aid of examples which do not, however, constitute a restriction of the inventive concept.
**FIG. 1** shows the catalytic hydrogenation of 4-iodonitrobenzene with NCNT and Pt/C and compares the % conversion, yield and selectivity.
**FIG. 2** shows the catalytic hydrogenation of 4-nitrobenzonitrile with NCNTs and 1% Pt/C and compares the % conversion, yield and selectivity.
**FIG. 3** shows the catalytic hydrogenation of ethyl 4-nitrocinnmate with NCNTs and 1% Pt/C and compares the % conversion, yield and selectivity.

### Example 1

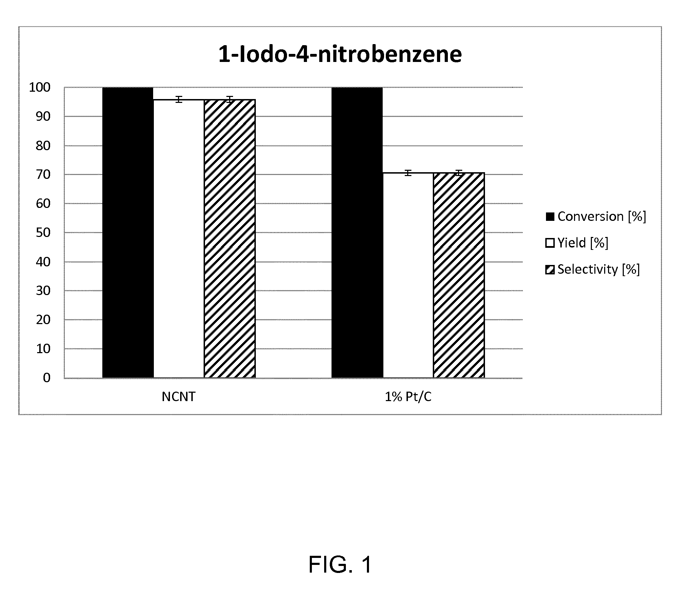

NCNTs were prepared as described in WO2009080204 A1 and WO2009149849 A1. In particular, the NCNTs were first washed with 10 % HCl solution to remove metal oxide impurities introduced during the NCNT synthesis stage. With regard to 4-iodonitrobenzene, the purified NCNTs demonstrated very high hydrogenation selectivity to 4-iodoaniline molecule under normal conditions (see Scheme 1). The reaction was carried out in the slurry type parallel reactors under a hydrogen pressure of 5-60 bar, temperature of 20-110 °C and reaction time of 10-300 minutes. In comparison with reference catalyst, Pt/C (Pt/C= Platin and Carbon, Johnson Matthey, Hertfordshire), NCNT catalysts showed higher hydrogenation selectivity and yield (see Fig. 1 and Reaction 1). These results suggest that the NCNT-based catalysts have the potential to be cost effective and metal-leaching-free alternatives for conventional Pt or Pd based catalysts in the field of halo nitro aromatics hydrogenation.

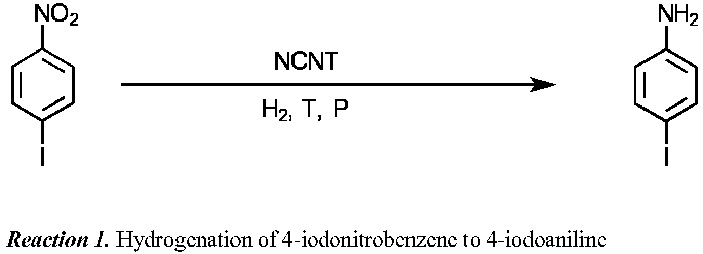

### Example 2

NCNTs were prepared as described in WO2009080204 A1 and WO2009149849 A1. In particular, the NCNTs were first washed with 10 % HCl solution to remove metal oxide impurities introduced during the NCNT synthesis stage. With regard to 4-iodonitrobenzene, 4-nitrobenzonitrile and ethyl 4-nitrocinnmate, the purified NCNTs demonstrated very high hydrogenation selectivity to the corresponding aniline derivatives under normal conditions (see Scheme 1). The reaction was carried out in the slurry type parallel reactors under a hydrogen pressure of 5-60 bar, temperature of 20-110 °C and reaction time of 10-300 minutes. In comparison with the reference catalyst, Pt/C (Pt/C= Platin and Carbon, Johnson Matthey, Hertfordshire), the NCNT catalysts showed higher hydrogenation selectivity and yield (see Fig. 1). These results suggest that the NCNT-based catalysts have the potential to be cost effective and metal-leaching-free alternatives for conventional Pt or Pd based catalysts in the field of functional nitro aromatics hydrogenation.

### Hydrogenation of 1-iodo-4-nitrobenzene

For the reference test 200 mg of 1-iodo-4-nitrobenzene and 60 mg of 5% Pt/C (Johnson Matthey) were weighed and filled in a 20 mL glass vial with a magnetic stirrer inside. 10 mL isopropanol were added as solvent. The filled glass vial was weighed and put in one of the 20 mL stainless steel reaction chambers of the 6-fold parallel-reactors. The reaction chamber was closed and a pressure test was carried out (The reactor was pressurized with 10 bar of nitrogen. After 10 min the pressure drop should not be bigger than 0.1 bar.). The pressure was released and the reactor was flushed two times with 4 bar of nitrogen. The reactor was heated up to 60 °C while stirring with a stirring rate of ∼ 800 rpm. Then the reactor was pressurized with 10 bar of hydrogen. The pressure decreased with time. After 30 min the remaining pressure (7.5 bar) was released and the reactor was flushed two times with 4 bar of nitrogen. The reactor was cooled down and the reactor chamber was opened. The glass vial was weighed again to determine the loss of solvent. The catalyst was isolated by filtrating using a membrane filter. 1 mL of the reaction mixture was diluted with 4 mL of isopropanol and was filled in a HPLC-vial. The analysis was carried out by HPLC (column: Agilent XDB-C18, 1.8 µm, 4.6x50 mm; method: see table 1). There was a complete conversion of 1-iodo-4-nitrobenzene. The selectivity to the target molecule 4-iodoaniline was 70.6%.

The experimental procedure for the test with NCNT (NCNT-MIV-11-E028 washed) was identical. 200 mg of 1-iodo-4-nitrobenzene and 120 mg of NCNT were used. The reaction temperature was 100 °C. The reaction time was 60 min. The initial hydrogen pressure was 40 bar, which decreased in 60 min at 35 bar. There was a complete conversion of 1-iodo-4-nitrobenzene. The selectivity to the target molecule 4-iodoaniline was 95.8%.

### Example 3 - Hydrogenation of 4-nitrobenzonitrile

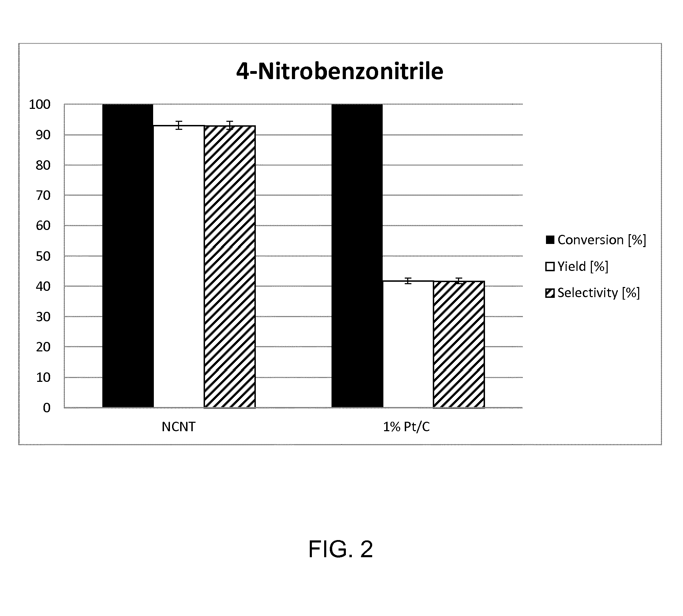

For the reference test 100 mg of 4-nitrobenzonitrile and 30 mg of 5% Pt/C (Johnson Matthey) were weighed and filled in a 20 mL glass vial with a magnetic stirrer inside. 10 mL isopropanol were added as solvent. The filled glass vial was weighed and put in one of the 20 mL stainless steel reaction chamber of the 6-fold parallel-reactors. The reaction chamber was closed and a pressure test was carried out (The reactor was pressurized with 10 bar of nitrogen. After 10 min the pressure drop should not be bigger than 0.1 bar.). The pressure was released and the reactor was flushed two times with 4 bar of nitrogen. The reactor was heated up to 60 °C while stirring with a stirring rate of ∼ 800 rpm. Then the reactor was pressurized with 10 bar of hydrogen. The pressure decreased with time. After 240 min the remaining pressure (7.7 bar) was released and the reactor was flushed two times with 4 bar of nitrogen. The reactor was cooled down and the reactor chamber was opened. The glass vial was weighed again to determine the loss of solvent. The catalyst was isolated by filtrating using a membrane filter. 1 mL of the reaction mixture was diluted with 4 mL of isopropanol and was filled in a HPLC-vial. The analysis was carried out by HPLC (column: Agilent XDB-C18, 1.8 µm, 4.6x50 mm; method: see Table 1, Scheme 2, Figure 2). There was a complete conversion of 4-nitrobenzonitrile. The selectivity to the target molecule 4-aminobenzonitrile was 42.2%.

The experimental procedure for the test with NCNT (NCNT-MIV-11-E028 washed) was identical. 100 mg of 4-nitrobenzonitrile and 60 mg of NCNT were used. The reaction temperature was 100 °C. The reaction time was 90 min. The initial hydrogen pressure was 40 bar, which decreased in 90 min at 34 bar. There was a complete conversion of 4-nitrobenzonitrile. The selectivity to the target molecule 4-aminobenzonitrile was 93.6%.

**Table 1: HPLC-method of the analysis of 1-iodo-4-nitrobenzene and 4-nitrobenzonitrile**

| | |
|---|---|
| flow | 2 mL/min |
| time | 10 min |
| temperature | 55 °C |
| injector volume | 2 µL |
| wavelength | 210 nm |
| flow agent A | phosphate buffer |
| flow agent B | acetonitrile |

| gradient | |
|---|---|
| time (min) | %B |
| 0 | 10 |
| 4.5 | 25 |
| 6.5 | 50 |
| 8 | 60 |
| 10 | 60 |

### Example 4 - Hydrogenation of ethyl 4-nitrocinnmate

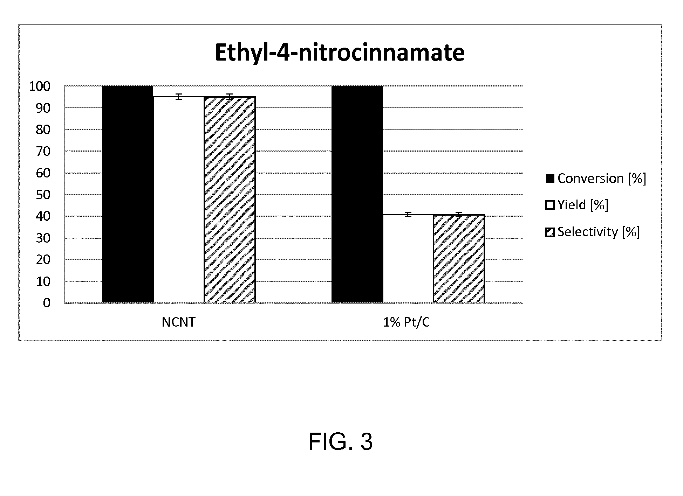

For the reference test 200 mg of ethyl 4-nitrocinnamate and 30 mg of 5% Pt/C (Johnson Matthey) were weighed and filled in a 20 mL glass vial with a magnetic stirrer inside. 10 mL isopropanol were added as solvent. The filled glass vial was weighed and put in one of the 20 mL stainless steel reaction chambers of the 6-fold parallel-reactors. The reaction chamber was closed and a pressure test was carried out (The reactor was pressurized with 10 bar of nitrogen. After 10 min the pressure drop should not be bigger than 0.1 bar.). The pressure was released and the reactor was flushed two times with 4 bar of nitrogen. The reactor was heated up to 60 °C while stirring with a stirring rate of ∼ 800 rpm. Then the reactor was pressurized with 10 bar of hydrogen. The pressure decreased with time. After 30 min the remaining pressure (6.5 bar) was released and the reactor was flushed two times with 4 bar of nitrogen. The reactor was cooled down and the reactor chamber was opened. The glass vial was weighed again to determine the loss of solvent. The catalyst was isolated by filtrating using a membrane filter. 1 mL of the reaction mixture was diluted with 4 mL of isopropanol and was filled in a HPLC-vial. The analysis was carried out by HPLC (column: Agilent XDB-C18, 1.8 µm, 4.6x50 mm; method: see Table 2, Scheme 2 and Figure 3). There was a complete conversion of ethyl 4-nitrocinnamate. The selectivity to the target molecule ethyl 4-aminocinnamate was 40.8%.

The experimental procedure for the test with NCNT (NCNT-MIV-11-E028 washed) was identical. 200 mg of ethyl 4-nitrocinnamate and 120 mg of NCNT were used. The reaction temperature was 100°C. The reaction time was 40 min. The initial hydrogen pressure was 40 bar, which decreased in 40 min at 34.5 bar. There was a complete conversion of ethyl 4-aminocinnamate. The selectivity to the target molecule p-iodoaniline was 95.2%.

**Table 2: HPLC-method of the analysis of ethyl 4-nitrocinnamate**

| | |
|---|---|
| flow | 2 mL/min |
| time | 12 min |
| temperature | 55 °C |
| injector volume | 2 µL |
| wavelength | 210 nm |
| flow agent A | phosphate buffer |
| flow agent B | acetonitrile |

| gradient | |
|---|---|
| time (min) | %B |
| 0 | 10 |
| 4.5 | 25 |
| 6.5 | 50 |
| .8 | 60 |
| 12 | 60 |

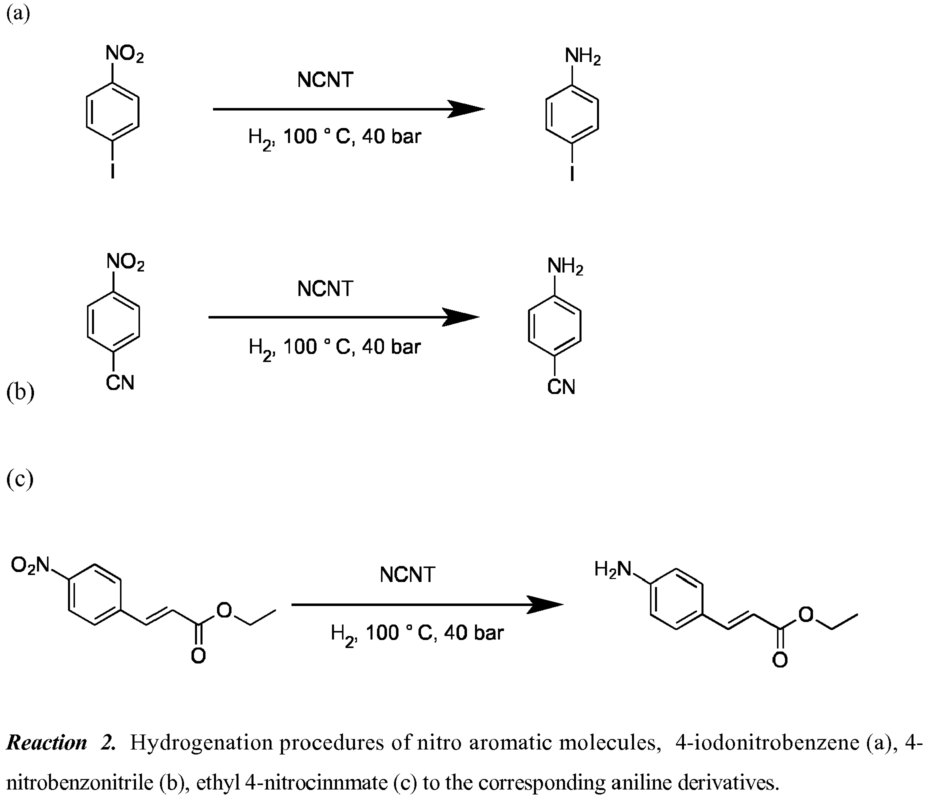

## Claims

1. A process for hydrogenating halogen-substituted nitro-aromatic compounds or functional nitro-aromatic compounds by means of hydrogen, **characterized in that** the hydrogenation is carried out in the presence of a catalyst which comprises nitrogen-doped carbon nanotubes as catalytically active component and, the proportion of nitrogen in the nitrogen-doped carbon nanotubes is in the range from 0.05 to 20% by weight in the graphitic layers and the nitrogen is present at least partly in a pyridinic arrangement.

2. The process as claimed in claim 1, **characterized in that** the halogen-substituted or functional nitro-aromatic compound is present in a liquid phase and the hydrogen in the present in gaseous form.

3. The process as claimed in claim 1, **characterized in that** the halogen-substituted or functional nitro-aromatic compound is present in a liquid solvent.

4. The process as claimed in claim 3, **characterized in that** the solvent is an organic solvent which does not comprise any hydrogenation-active functional group or is a mixture of such organic solvents.

5. The process as claimed in any of claims 2 to 4, **characterized in that** it is carried out at temperatures in the range from 20°C to 350°C, preferably from 40°C to 110°C.

6. The process as claimed in claim 1, **characterized in that** the halogen-substituted or functional nitro-aromatic compounds and the hydrogen are present in gaseous form.

7. The process as claimed in any of claims 1 to 6, **characterized in that** it is carried out in a plurality of stages, preferably in from 2 to 10, particularly preferably from 2 to 6, very particularly preferably from 2 to 5, in particular 2 or 3, reaction zones connected in series.

8. The process as claimed in any of claims 1 to 7, **characterized in that** the catalyst contains at least 50% by weight, preferably at least 80% by weight, particularly preferably at least 95% by weight, of nitrogen-doped carbon nanotubes.

9. The process as claimed in any of claims 1 to 8, **characterized in that** the catalyst is present as a fixed bed.

10. The process as claimed in claim 9, **characterized in that** the fixed bed is present in the form of a structured bed in which the catalyst activity increases in the main flow direction in the reaction zone.

11. The process as claimed in any one of claims 1 to 10, **characterized in that** the halogen-substituted nitro-aromatic compound is selected from the group consisting of 4-Iodo-nitrobenzene, 4-Fluoronitrobenzene, 4-Chloronitrobenzene, 4-Bromonitrobenzene, 1-Chloro-3-nitrobenzene, 1-Bromo-4-chloro-2-nitrobenzene, 5-Chloro-2-nitroaniline, 4-Chloro-3-nitroanisole, 2-Chloro-4-nitrotoluene, 1-Bromo-4-nitrobenzene, 2-Bromo-1-tert-butyl-4-nitrobenzene, 1-Iodo-2,4-dimethyl-6-nitrobenzene, 1-Chloro-2-iodo-5-nitrobenzene and 2-Iodo-3-nitrotoluene.

12. The process as claimed in any one of claims 1 to 10, **characterized in that** the functional nitro-aromatic compound is selected from the group consisting of 4-Nitrobenzonitrile, Ethyl 4-nitrocinnmate, 4-Nitrocinnamic acid, 4-Nitrocinnamyl alcohol, 2-Chloro-4-nitrobenzonitrile, 4-Amino-3-nitrobenzonitrile, 4-Hydroxy-3-nitrobenzonitrile, 4-Bromo-3-nitrobenzonitrile, (1,4-dihydro-6-nitro)-1,4-Methanonaphthalene and 1-(2-cyclopropylethenyl)-2-nitro-Benzene.
